# EUROPEAN PATENT APPLICATION

(11) **EP 4 571 772 A1**
(43) Date of publication of application: **18.06.2025**
(21) Application number: 23216516.7
(22) Date of filing: 14.12.2023
(51) Int. Cl.: G16H 40/63

(54) **INSTRUCTION METHOD ADJUSTED USING SUBJECT DATA**

(71) Applicant: Koninklijke Philips N.V., 5656 AG Eindhoven (NL)
(72) Inventor: WESTERINK, Joanne Henriëtte Desirée Monique, Eindhoven (NL); ANAND, Shreya, Eindhoven (NL); MUIJLWIJK, Heleen, 5656AG Eindhoven (NL); DIAZ MENDOZA, Jose, Eindhoven (NL); MENA BENITO, Maria Estrella, Eindhoven (NL); VAN EE, Raymond, Eindhoven (NL)
(74) Representative: Philips Intellectual Property & Standards

(57) **Abstract**

Disclosed herein is a medical system (100, 300) comprising a memory (110) storing machine executable instructions (130) and an instruction database (132) storing instruction sections indexed by instruction type (138) and instruction content (136). The medical system further comprises a presentation system (118) for displaying instructions (120) to a subject (112) and a subject data collection system (114) providing subject data (134, 144). Execution of the machine executable instructions causes a computational system (104) to: receive (204) a list of instruction content (136); receive (206) initial subject data (134) from the subject data collection system; and determine (208) the instruction type using the initial subject data. Execution of the machine executable instructions causes the computational system to repeatedly: retrieve (210) a next instruction section (140) from the instruction database; present (212) the next instruction section using the presentation system; and adjusting (214) the instruction type using the updated subject data.

## Description

### TECHNICAL FIELD

The invention relates to providing instructions using a medical system.

### BACKGROUND

Communication can best be summarized as the transmission of a message from a sender to a receiver in an understandable manner. Effective communication means sharing of a common meaning between the sender and receiver, that is, effective communication leads to understanding the meaning of the message how it is intended by the sender.

Instructions are one type of communication. Medical systems such as complex medical imaging system may be difficult to operate or understand. Often times if detailed instructions are provided to a subject, the subject may still have difficulty operating or repairing the medical system.

### SUMMARY

The invention provides for a medical system, a computer program, and a method in the independent claims. Embodiments are given in the dependent claims.

In one aspect the invention provides for a medical system that comprises a memory storing machine-executable instructions and an instruction database. The instruction database comprises instruction sections indexed by instruction type and instruction content. The medical system further comprises a presentation system configured for displaying instructions to a subject. The medical system further comprises a subject data collection system configured for collecting subject data descriptive of the subject.

The medical system further comprises a computational system. Execution of the machine-executable instructions causes the computational system to receive a list of instruction content. Execution of the machine-executable instructions further causes the computational system to receive initial subject data from the subject data collection system. Execution of the machine-executable instructions further causes the computational system to determine the instruction type using the initial subject data.

Execution of the machine-executable instructions further causes the computational system to repeatedly retrieve a next instruction section from the instruction database by querying the instruction database with the instruction type and a next instruction content selected from the list of instruction content. Execution of the machine-executable instructions further causes the computational system to repeatedly present the next instruction section using the presentation system. Execution of the machine-executable instructions further causes the computational system to repeatedly, in case of receiving updated subject data from the subject data collection system, adjusting the instruction type using the updated subject data.

In another aspect the invention provides for a method. The method comprises receiving a list of instruction content. The method further comprises receiving initial subject data from a subject data collection system. The subject data collection system is configured for collecting subject data descriptive of a subject. The method further comprises determining the instruction type using the initial subject data.

The method further comprises repeatedly retrieving a next instruction section from the instruction database by querying an instruction database with the instruction type and a next instruction content selected from the list of instruction content. The instruction database comprises instruction sections indexed by the instruction type and instruction content. The method further comprises repeatedly presenting the next instruction section using a presentation system. The presentation system is configured for displaying instructions to the subject. The method further comprises repeatedly adjusting the instruction type using the updated subject data if updated subject data is received from the subject data collection system.

In another aspect, the invention provides for a computer program comprising machine-executable instructions. Execution of the machine-executable instructions causes a computational system to receive a list of instruction content. Execution of the machine-executable instructions further causes the computational system to receive initial subject data from a subject data collection system. The subject data collection system is configured for collecting subject data descriptive of a subject. Execution of the machine-executable instructions further causes the computational system to determine the instruction type using the initial subject data.

Execution of the machine-executable instructions further causes the computational system to repeatedly retrieve a next instruction section from the instruction database by querying an instruction database with the instruction type and a next instruction content selected from the list of instruction content. The instruction database comprises instruction sections indexed by instruction type and instruction content. Execution of the machine-executable instructions further causes the computational system to repeatedly present the next instruction section using a presentation system. The presentation system is configured for displaying instructions to the subject. Execution of the machine-executable instructions further causes the computational system to repeatedly adjust the instruction type using the updated subject data if updated subject data is received from the subject data collection system.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 3.

### DESCRIPTION OF EMBODIMENTS

Like numbered elements in these figures are either equivalent elements or perform the same function. Elements which have been discussed previously will not necessarily be discussed in later figures if the function is equivalent.

In an example, a medical system comprises a memory storing machine-executable instructions and an instruction database. An instruction database as used herein may encompass a variety of ways of storing information. For example, the instruction database could be a table. In other examples the instruction database is a relational database. In yet further examples, the instruction database may be a collection of data or files stored in a memory or memory system. The instruction database comprises instruction sections indexed by instruction type and instruction content. In some examples, the instruction type may be descriptive of the type of instruction or the modality used for the instruction. For example, the particular instruction type may be oriented or geared towards a particular type of learning. The instruction content may refer to technical or instructional data which is contained by a particular data record. In some examples, the instructions in the instruction database comprise instructions or commands on how to perform or do a task. In other examples, the data in the instructional database may be more theoretical. For example, the instructions in the instructional database may contain theoretical data which may be learned by a subject.

The medical system further comprises a presentation system configured for displaying instructions to a subject. The presentation system may take different forms in different examples. The presentation system may for example be a screen or monitor of a computer system. In other examples, it may be for example the screen or touchscreen of a portable computing device or a mobile telecommunications device such as a smartphone.

The medical system further comprises a subject data collection system configured for collecting subject data descriptive of the subject. The subject data collection system may take different forms in different examples. In some examples, the subject data collection system may monitor the behavior or activities of the subject. In yet other examples, the subject data collection system may perform physiological or physical measurements on the subject. For example, it may monitor the blood pressure, blood oxygenation, the conductivity of the skin or other properties.

The medical system further comprises a computational system. Execution of the machine-executable instructions causes the computational system to receive a list of instruction content. This for example, may be a list of topics or instruction sections which are desired to be presented to the subject. The list of instruction content may in some examples be a combination of commands or instructions which enable the user to perform a task. In yet other examples, the list of instruction content may be for example a curriculum or list of theoretical knowledge. Execution of the machine-executable instructions further causes the computational system to receive initial subject data from the subject data collection system. Execution of the machine-executable instructions further causes the computational system to determine the instruction type using the initial subject data. The instruction type is therefore personalized to the subject using the initial subject data.

Execution of the machine-executable instructions further causes the computational system to repeatedly retrieve a next instruction section from the instruction database by querying the instruction database with the instruction type and a next instruction content selected from the list of instruction content. The instruction type has been determined from the subject data or initial subject data and therefore is used to personalize or choose the next instruction content which is appropriate for a particular subject. Execution of the machine-executable instructions further causes the computational system to repeatedly present the next instruction section using the presentation system. After the next instruction section from the instruction database has been retrieved, it is then presented to the subject.

Execution of the machine-executable instructions further causes the computational system to repeatedly, in case of receiving updated subject data from the subject data collection system, adjusting the instruction type using the updated subject data. In this example, the system forms a closed control loop. The instruction type is determined using the initial subject data. However, if during the course of presenting the next instruction to the subject there is a change in the subject data as measured by the subject data collection system, the instruction type may be adjusted. This for example, may be very useful in adjusting the content or instructions which is presented to the person based on their physiological response or other physical or mental response.

Examples may have the benefit of providing for a more effective method of presenting instructions to a subject. This is because using the subject data to determine the instruction type provides a closed feedback loop. The initial subject data is used to select the instruction type, and as next instructions sections are presented updated subject data may be received to update the instruction type before the instruction database is queried again. The instructions presented to the subject are constantly adapted.

In another example the subject data collection system comprises a subject sensor system. The subject sensor system as used herein may encompass a system which is able to perform a measurement or acquire data which is descriptive of the subject.

In another example, the subject data comprises subject eye motion data descriptive of reading the presentation system by the subject.

The eye tracking system may for example be a commercially available system which measures where a user is looking or their point of gaze. Such systems are known and may operate using different principles such as an attachment to the eye such as an embedded mirror or magnetic sensor. Other eye tracking systems may use non-contact optical measurements of eye motion.

In another example the subject data comprises the subject pupil width. For example, there may be a camera system which measures the subject pupil width. The subject pupil width may for example be a measure of how engaged or interested the subject is.

In another example, the subject data comprises subject skin conductivity. There may for example be a system which measures the skin conductivity as part of the subject sensor system.

In another example, the subject sensor system further comprises subject physical activity. For example, the subject may have a wearable accelerometer which is used for measuring the activity of the subject.

In another example, the subject data comprises a subject average physical activity. This may for example be the average physical activity over a predetermined period of time.

In another example, the subject data further comprises the subject heart rate. The subject sensor system may for example encompass a heart rate monitor.

In another example, the subject data comprises the subject blood pressure. The subject sensor system may comprise a system for measuring the subject blood pressure.

In another example, the subject data further comprises a subject blood oxygen level. For example, the subject sensor system may comprise a wearable blood oxygen level meter.

In another example, the subject data further comprises the subject surrounding noise environment classification. For example, there may be a microphone which is on a wearable device or device adjacent to the subject, which is able to measure the surround noise environment and then classify it. For example, the classification may be performed by a classifier neural network.

In another example, the subject data further comprises a subject speech volume level. The subject sensor system may for example comprise a microphone and a digitization system for measuring the subject speech and then determining its volume level.

In another example, the subject data further comprises a detection of subject swearing. This may include remarks or comments by the subject which indicate a level of agitation. The subject sensor system may for example encompass a system for digitizing the speech of the subject and then have a speech-to-text module. The text may then for example be compared against a list of keywords.

In another example, the subject data further comprises detection of subject singing. As with the subject swearing, there may be a speech-to-text system which is used to detect phrases which may be commonly used in singing. It may also monitor the pitch of the subject's voice and determine if it is fluctuating more than would be common for speaking.

In another example, the subject data further comprises a duration of a subject reading a text presented by the presentation system. For example, this may be monitoring how long a subject reads a page or how long the subject is viewing a particular section of text. This may for example be useful in measuring the level of concentration or ability of the subject to concentrate at the time when the subject data is measured.

In another example, the subject data further comprises face gestures of the subject. There may for example be a camera system and then a neural network configured to classify the face gestures.

The various examples of subject data may be beneficial because they may provide for an objective means of measuring the current state of the subject and depending upon the subject data, the appropriate instruction type may be selected. For example, if the subject is having a hard time concentrating and this is indicated by the duration of the subject reading the text being relatively small compared to a threshold value, then it may be beneficial to present video instructions as opposed to having the subject read particular instructions.

In another example, the subject data comprises the times the instruction section was started. For example, the instructions may be presented to the subject and then the subject restarts or begins these instructions from the beginning. This may for example indicate that the subject is not receptive to the particular type of instructions being presented or is having a hard time concentrating on them.

In another example, the subject data further comprises the times the instruction section was repeated. If the subject needs to repeat the instruction section multiple times, this may for example indicate that they are not effective for communicating or providing the instructions to the subject.

In another example, the subject data further comprises the times the instruction section was paused. If the subject needed to pause the instructions or delay them, this may indicate that the subject had a hard time understanding them or they were not effective in providing instructions.

In another example, the subject data further comprises the times additional information was accessed when the instruction section was presented. This again may indicate that the subject data or instructions presented were not effective in instructing the subject.

In another example, the subject data further comprises the reaction time when prompted to start a next instruction section. This may for example be an objective measure of whether the subject is paying attention to or is able to interpret the instruction section.

In another example, the instruction type is at least partially determined using a historical subject preference. These may for example be a subject preference which was pre-programmed or used for a particular subject before. Execution of the machine-executable instructions further causes the computational system to preferably modify the historical subject preference using the updated subject data. In this example, updating the subject preference using the updated subject data may for example be useful in changing the historical subject preference to a classification or value which is more appropriate for the subject.

In another example, the instruction type is at least partially determined using a trained instruction type machine learning module configured to provide the instruction type in response to receiving the initial subject data and/or the updated subject data as input. This may for example be an effective means of using the initial subject data or the updated subject data to control the instruction type.

The trained instruction type machine learning module may for example be a neural network. A convolutional neural network or a neural network with several fully connected layers may be used and may be effective for this task. In general, neural networks which are used for providing classifications may be used. The neural network may be trained by collecting the subject data and then manually labeling it by a person. This may for example be used in a deep learning procedure to train the neural network.

In another example, execution of the machine-executable instructions further causes the computational system to adjust an order of the instruction sections by comparing the updated subject data to a predetermined adjustment criterion. In this example, the predetermined adjustment criterion may be a set of rules which control how the updated subject data is used to rearrange the order of the instruction sections. For example, the type of data or media which is presented may affect the order in which they are presented. This may have the effect of providing more effective instructions to the subject or enable the subject to perform the instructions more effectively.

In another example, execution of the machine-executable instructions further causes the computational system to trigger an information query if the updated subject data meets a predetermined query condition. Execution of the machine-executable instructions further causes the computational system to receive an additional instruction section in response to querying an information source with the information query. Execution of the machine-executable instructions further causes the computational system to present the additional instruction section using the presentation system. The updated subject data may for example indicate that the subject is unable to follow the instruction section. For example, if one of the measurements in the updated subject data is outside of the predetermined range or condition then this may trigger the information query. The information query may for example be data that is retrieved from a database, may be retrieved from an internet search, or may even be provided by a question coded in the predetermined query condition to a large language model. This response to querying the information source may then for example be presented as additional instructions on using the presentation system. This may have the benefit of providing additional information on the fly if their predetermined query condition is met.

In another example, execution of the machine-executable instructions further causes the computational system to determine an added section score by comparing subject data collected during presentation of the additional instruction section by the presentation system with a predetermined score criterion. Execution of the machine-executable instructions further causes the computational system to add the additional instruction section to the instruction database if the added section score meets a predetermined score criterion. For example, if the subject data presented during the additional instruction section indicates that it suddenly meets the predetermined query condition as described above, then this may indicate that the material is appropriate or is easier for the subject to follow. In this case, this may then be added to the database so that it may be used for additional queries for future instructions.

In another example, execution of the machine-executable instructions further causes the computational system to query the subject with a quiz or self-assessment after presentation of the next instruction section. Execution of the machine-executable instructions further causes the computational system to determine a score of the query or a self-assessment. The updated subject data comprises the score. In this example, the self-assessment or quiz is used as part of the data which is used to determine the updated subject data.

In another example the list of instructions content comprises medical instructions. These, for example may be instructions which an assistant could perform to a subject. They may also be instructions which should be performed in an emergency. For example, they may be instructions provided to an untrained individual to deal with a medical incident or emergency. In other examples, they may be a checklist of procedures or tasks which should be performed during a medical procedure or inspection. For example, the list of instruction content may contain instructions or a checklist for actions to be performed before or after a medical procedure. In other examples, the medical instructions may be procedures used for operating or setting up a medical imaging system.

In another example, the list of instruction contents comprises a repair procedure of a medical imaging system. In another example, the list of instruction contents comprises an operating procedure of the medical imaging system. In both of these cases it may be beneficial because they may provide for detailed steps to an untrained or trained individual to perform during repairing or operating a medical imaging system.

In some examples the medical imaging system may be a tomographic medical imaging system. In other examples, it may be a positron emission tomography system, a single-photon emission tomography system, a computed tomography system, an ultrasound system, or a magnetic resonance imaging system. In other examples there may be a combination of two or more of these imaging modalities.

In another example, the medical system further comprises the medical imaging system. The subject data collection system is configured to query the medical imaging system for a change in the machine status during the presentation of the instruction sections by the presentation system. The updated subject data comprises the change in the machine status. The instruction type is at least partially adjusted using the change in the machine status. In this example, the updated subject data is based on the actual machine state of the medical imaging system. For example, this may be used to monitor a repair procedure or operation procedure of the medical imaging system. Based on the actions taken on the individual medical imaging system, further instructions are then adapted and presented to the individual.

In another example, execution of the machine-executable instructions further causes the computational system to receive an instruction topic. Execution of the machine-executable instructions further causes the computational system to determine the list of instruction content by retrieving the list of instruction content from a list database using the instruction topic or by receiving the list of instruction content in response to inputting the instruction topic into a trained instruction topic machine learning module. In this example, an instruction topic is received and then it is either provided by using a database or from a trained machine learning system or module. For example, a large language model could be queried which could provide the list of instruction content. This may for example provide a series of instructions or educational material to fulfill the instruction topic.

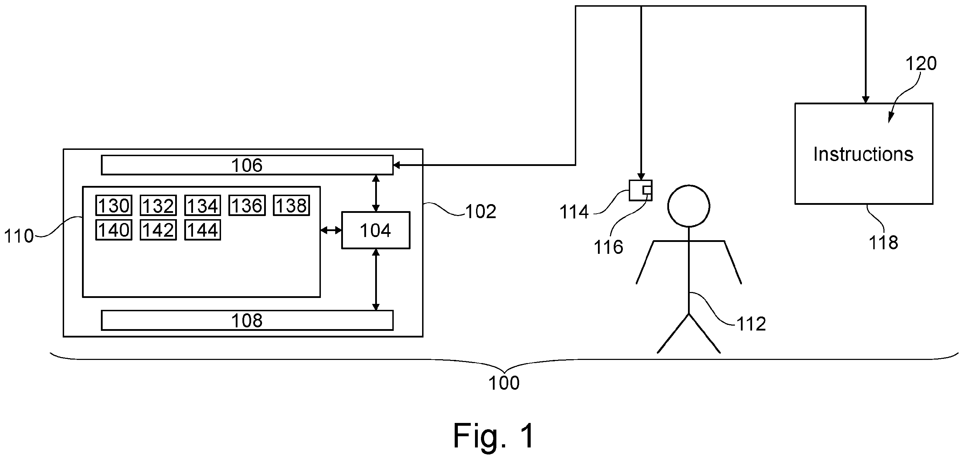

Fig. 1 illustrates an example of a medical system 100. The medical system 100 is shown as comprising a computer 102. The computer 102 may represent one or more computers at one or more locations. The computer 102 is shown as comprising a computational system 104. Likewise, the computational system 104 may represent one or more computational systems or computing cores located at one or more locations.

The computational system 104 is shown as being in communication with a hardware interface 106, an optional user interface 108, and a memory 110. The memory 110 is intended to represent various types of memory which are accessible to the computational system 104. In some examples the memory 110 comprises or is a non-transitory storage medium.

The memory 110 is shown as containing machine-executable instructions 130. The machine-executable instructions 130 enable the computational system 104 to perform basic data and text manipulation tasks as well as control and operate other components of the medical system 100 via the hardware interface 106. The hardware interface 106 is shown as being in communication with a subject data collection system 114 and a presentation system 118. The subject data collection system 114 is able to collect subject data and in this example, it is shown as optionally comprising a subject sensor system 116 for performing a physical measurement on the subject 112.

In various examples, the subject data could comprise, but is not limited to, any one of the following: subject eye motion data descriptive of reading the presentation system by the subject, subject pupil width, subject skin conductivity, subject physical activity, subject average physical activity, subject heart rate, subject blood pressure, subject blood oxygen level, subject surrounding noise environment classification, subject speech volume level, detection of subject swearing, detection of subject singing, duration of subject reading a text presented by the presentation system, face gestures of the subject, and combinations thereof.

The presentation system 118 may be visible to the subject 112 and may present instructions 120 in the form of instruction steps to follow or instructional material. The medical system 100 forms a closed control loop. The presentation system 118 is able to present instructions 120 to the subject 112. The subject data collection system 114 then collects subject data descriptive of the subject 112 which is used to modify the instructions 120.

The memory 110 is additionally shown as containing an optional instruction topic 132. The instruction topic 132 could for example be a task such as to perform a medical task or to perform a repair, or an instructional topic. The memory 110 is further shown as containing an optional list database 134 that contains a list of instruction content 136 referenced or indexed by the instruction topic 132. The list of instruction content 136 is a list of various topics or modules or instructions that should be presented to the subject 112. The memory 110 is further shown as containing an instruction database 132. The instruction database 132 contains instruction sections that are referenced as a function of the instruction type and the instruction content. The memory 110 is shown as containing initial subject data 134 that has been collected by the subject data collection system 114. The memory 110 is further shown as containing an optional neural network or rule module 136 that is able to receive the subject data as input and to output an instruction type 138.

The optional neural network may for example provide a classification of the subject data or the measurements contained in the subject data. The memory 110 is shown as containing this instruction type 138 that has been obtained by inputting the initial subject data 134 into the optional neural network 136 or rule module. The memory 110 is further shown as containing a next instruction content 142 that has been selected from the list of instruction content 136. They may for example be selected sequentially. The memory 110 is further shown as containing a next instruction section that has been retrieved from the instruction database 132 by querying it with the instruction type 138 and the next instruction content 142. The instructions 120 presented on the presentation system 118 may be a rendering of the next instruction section 140.

Examples may comprise an instruction database 132 storing instruction sections 142 that contain various lectures, trainings, or detailed instructions. Each instruction 142 section may delivered as a train of pre-recorded smaller sections, and each of those presents a kernel of knowledge that is important to the subjects. A lecture or training can be composed by combining the relevant sections in a sequence conforming to the learning goals of the lecture/training. The subject's understanding can be assessed by means of an exam or a quiz at the end of the training, but it is also a possibility to include a number of intermediate quizzes in between consecutive sections of the presentation sequence.

However, a one-lecture-fits-all type of training cannot be optimal for all subjects, as it is known that subjects differ in their learning style, for instance in the type of content elaboration they prefer: some subjects might be best helped by a range of examples to grasp the impact of a certain kernel of knowledge, while other subjects would gain more insight through a formula that would present the knowledge in a more abstract form.

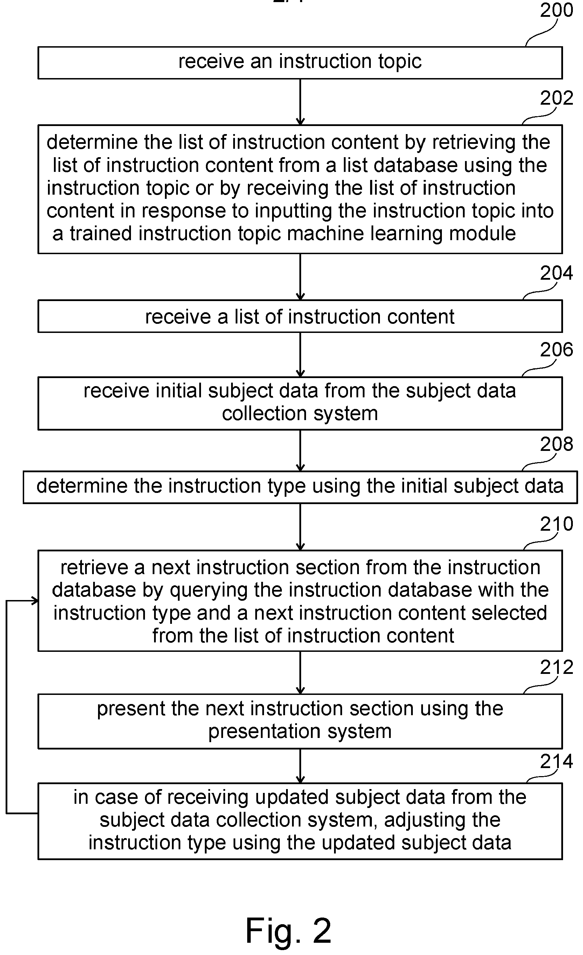

Fig. 2 shows a flow chart which illustrates a method of operating the medical system of Fig. 1. In step 200 an instruction topic 132 is optionally received. The instruction topic 132 may be optionally used to retrieve a list of instruction content 136 from a list database 134.

In step 204 the list of instruction content 136 is received. This may be done using the list database 134 as described above or in some examples, the list of instruction content 136 may be directly provided or retrieved from a file or storage location in the memory 110. In step 206 the initial subject data 134 is received from the subject data collection system 114. The instruction type 138 is then determined using the initial subject data 134. An optional neural network or rule module 136 may be used to classify the initial subject data 134. The classification may be the instruction type 138.

In step 210, a next instruction section 140 is retrieved from the instruction database by querying the instruction database 132 with the instruction type 138 as a next instruction content 142 selected from the list of instruction content 136. In some examples, the list of instruction content 136 may be an ordered list that contains the instruction in an order or sequence.

In step the next instruction section 140 is presented using the presentation system 118. The next instruction section 140 may contain instruction steps to perform and/or educational material which may be presented on the presentation system 118 in the form of instructions 120. In step 214, if updated subject data 144 is received from the subject data collection system 114 then the instruction type 138 may be updated using this updated subject data 144. The method then may proceed back to step 210 until all instruction sections corresponding to the list of instruction content have been presented. The loop formed by steps 210, 212, and 214 forms a closed control loop.

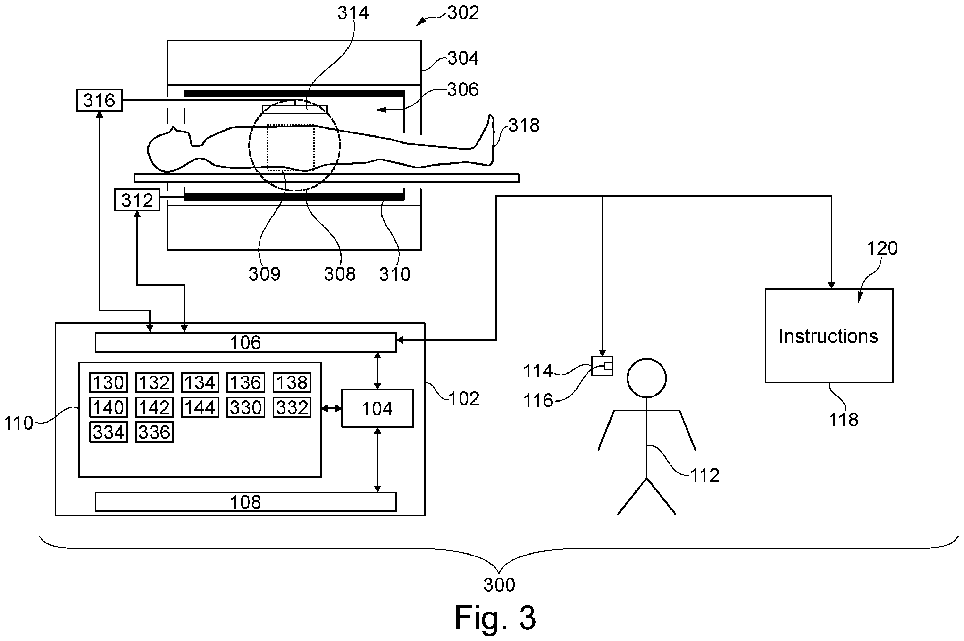

Fig. 3 illustrates a further example of a medical system 300. The medical system 300 is similar to the medical system 100 presented in Fig. 1, except that it additionally comprises a magnetic resonance imaging system 302. The magnetic resonance imaging system 302 is an example of a medical imaging system. The magnetic resonance imaging system 302 may for example be replaced with other types of medical imaging systems such as a computed tomography system, a digital fluoroscope, a digital x-ray system, a positron emission tomography system, a single photon emission tomography system, and an ultrasound system.

The magnetic resonance imaging system 302 comprises a magnet 304. The magnet 304 is a superconducting cylindrical type magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data are acquired for the field of view 309. The region of interest could be identical with the field of view 309 or it could be a sub volume of the field of view 309. An examination subject 318 is shown as being supported by a subject support 320 such that at least a portion of the examination subject 318 is within the imaging zone 308 and the field of view 309.

Within the bore 306 of the magnet there is also a set of magnetic field gradient coils 310 which is used for the acquisition of measured k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The memory 110 is further shown as containing a machine state 330 that was obtained from the magnetic resonance imaging system 302 while the instructions 120 were presented on the presentation system 118. There may be several different usage cases. In one case the subject 112 could be repairing the magnetic resonance imaging system 302. The instructions 120 could be detailed instructions for the subject 112 to follow during the repair of the magnetic resonance imaging system 302. The machine state 330 may be used to properly update the next instruction section 140. This may provide for a more effective and complete repair of the magnetic resonance imaging system.

In another usage case, the instructions 120 could be instructions on how to set up and/or perform a magnetic resonance imaging protocol using the magnetic resonance imaging system 302. For example, the machine state 330 could be indicative of the configuration of the magnetic resonance imaging system. The feedback loop formed by the medical imaging system 300 could then be used to effectively set up and configure the magnetic resonance imaging system 302 for use.

The memory 110 is further shown as containing pulse sequence commands 332. The pulse sequence commands 332 are commands or data which may be converted into commands to control the magnetic resonance imaging system to acquire k-space data. The memory 110 is further shown as containing k-space data 334 that has been acquired by controlling the magnetic resonance imaging system 302 with the pulse sequence commands 332. The memory 110 is further shown as containing a magnetic resonance image 336 that has been reconstructed from the k-space data 334.

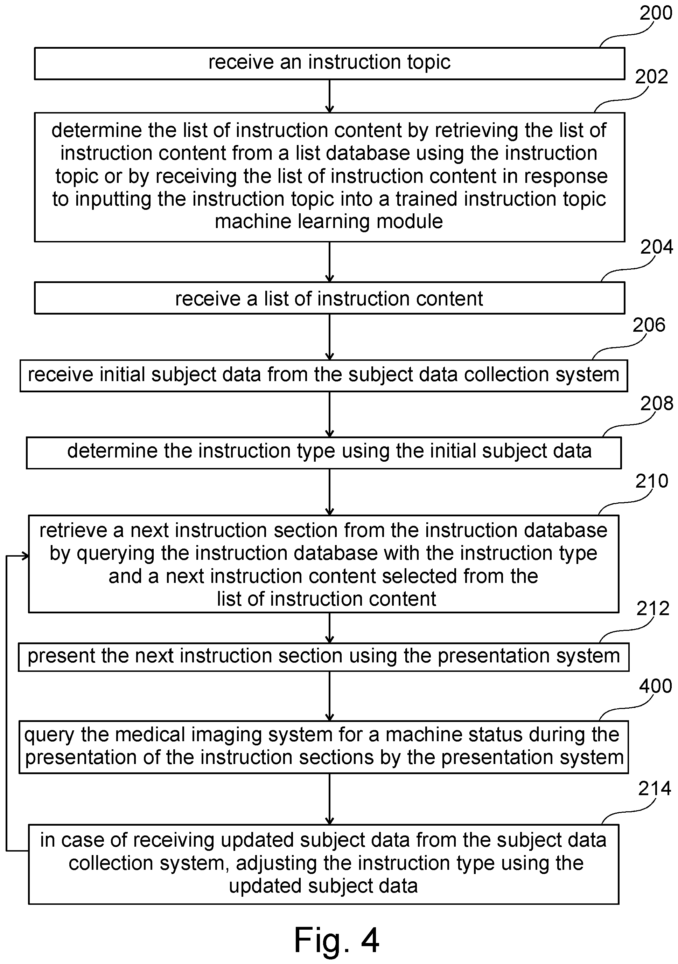

Fig. 4 shows a flowchart which illustrates a method of operating the medical system 300 of Fig. 3. Steps 200, 202, 204, 206, 208, 210, and 212 are performed as they were in Fig. 2. In step 400, the medical imaging system is queried for a machine status 330 during the presentation of the instruction sections, or in this case, the next instruction section 140 by the presentation system 118. The machine status 330 may then be combined with the updated subject data 144. As is described in 214 in change of the updated subject data 144 the instruction type 138 may be modified. As was mentioned above, this may be useful in modifying the form of the instructions 120 presented to the subject 112 so that the magnetic resonance imaging system 302 may be more effectively repaired or operated.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

100 medical system
102 computer
104 computational system
106 hardware interface
108 user interface
110 memory
112 subject
114 subject data collection system
116 subject sensor system
118 presentation system
120 instructions
130 machine executable instructions
132 instruction topic
134 list database
136 list of instruction content
132 instruction database
134 initial subject data
136 neural network or rule module
138 instruction type
140 next instruction section
142 next instruction content
144 updated subject data
200 receive an instruction topic
202 determine the list of instruction content by retrieving the list of instruction content from a list database using the instruction topic or by receiving the list of instruction content in response to inputting the instruction topic into a trained instruction topic machine learning module
204 receive the list of instruction content
206 receive initial subject data from the subject data collection system
208 determine the instruction type using the initial subject data
210 retrieve a next instruction section from the instruction database by querying the instruction database with the instruction type and a next instruction content selected from the list of instruction content
212 present the next instruction section using the presentation system
214 in case of receiving updated subject data from the subject data collection system, adjusting the instruction type using the updated subject data
300 medical system
302 magnetic resonance imaging system
304 magnet
306 bore of magnet
308 imaging zone
309 field of view
310 magnetic field gradient coils
312 magnetic field gradient coil power supply
314 radio frequency coil
316 transceiver
318 examination subject
320 subject support
330 machine status
332 pulse sequence commands
334 k-space data
336 magnetic resonance image
400 query the medical imaging system for a machine status during the presentation of the instruction sections by the presentation system

## Claims

1. A medical system (100, 300) comprising:
- a memory (110) storing machine executable instructions (130) and an instruction database (132), wherein the instruction database comprises instruction sections indexed by instruction type (138) and instruction content (136);
- a presentation system (118) configured for displaying instructions (120) to a subject (112);
- a subject data collection system (114) configured for collecting subject data (134, 144) descriptive of the subject;
- a computational system (104), wherein execution of the machine executable instructions causes the computational system to:
- receive (204) a list of instruction content (136);
- receive (206) initial subject data (134) from the subject data collection system; and
- determine (208) the instruction type using the initial subject data; wherein execution of the machine executable instructions causes the computational system to repeatedly:
- retrieve (210) a next instruction section (140) from the instruction database by querying the instruction database with the instruction type and a next instruction content selected from the list of instruction content;
- present (212) the next instruction section using the presentation system;
- in case (214) of receiving updated subject data (144) from the subject data collection system, adjusting the instruction type using the updated subject data.

2. The medical system of claim 1, wherein the subject data collection system comprises a subject sensor system (116), wherein the subject data comprises any one of the following: subject eye motion data descriptive of reading the presentation system by the subject, subject pupil width, subject skin conductivity, subject physical activity, subject average physical activity, subject heart rate, subject blood pressure, subject blood oxygen level, subject surrounding noise environment classification, subject speech volume level, detection of subject swearing, detection of subject singing, duration of subject reading a text presented by the presentation system, face gestures of the subject, and combinations thereof.

3. The medical system of claim 1 or 2, wherein the subject data comprises any one of the following: times the instruction section was started, times the instruction section was repeated, times the instruction section was paused, times additional information was accessed when the instruction section was presented, reaction time when prompted to start next instruction section, and combinations thereof.

4. The medical system of claim 1, 2, or 3, wherein the instruction type is at least partially determined using a historical subject preference, and wherein execution of the machine executable instructions further causes the computational system to preferably modify the historical subject preference using the updated subject data.

5. The medical system of any one of the preceding claims, wherein the instruction type is at least partially determined using a trained instruction type machine learning module configured to provide the instruction type in response to receiving the initial subject data or the updated subject data as input.

6. The medical system of any one of the preceding claims, wherein execution of the machine executable instructions further causes the computational system to adjust an order of the instruction sections by comparing the updated subject data to a predetermined adjustment criterion.

7. The medical system of any one of the preceding claims, wherein execution of the machine executable instructions further causes the computational system to:
- trigger an information query if the updated subject data meets a predetermined query condition;
- receive an additional instruction section in response to querying an information source with the information query; and
- present the additional instruction section using the presentation system.

8. The medical system of claim 7, wherein execution of the machine executable instructions further causes the computational system to:
- determine an added section score by comparing subject data collected during presentation of the additional instruction section by the presentation system with a predetermined score criterion; and
- add the additional instruction section to the instruction database if the added section score meets a predetermined score criterion.

9. The medical system of any one of the preceding claims, wherein execution of the machine executable instructions further causes the computational system to:
- query the subject with a quiz or self-assessment after presentation of the next instruction section; and
- determine a score of the quiz or self-assessment, wherein the updated subject data comprises the score.

10. The medical system of any one of the preceding claims, wherein the list of instruction content comprises medical instructions.

11. The medical system of any one of the preceding claims, wherein the list of instruction content comprises any one of the following: a repair procedure of a medical imaging system and an operation procedure of the medical imaging system.

12. The medical system of claim 11, wherein the medical system further comprises the medical imaging system, wherein the subject data collection system is configured to query (400) the medical imaging system for a machine status during the presentation of the instruction sections by the presentation system, wherein the updated subject data comprises the change in machine status, and wherein the instruction type is at least partially adjusted using the change in the machine status.

13. The medical system of any one of the preceding claims, wherein execution of the machine executable instructions further causes the computational system to:
- receive (200) an instruction topic; and
- determine (202) the list of instruction content by retrieving the list of instruction content from a list database (134) using the instruction topic or by receiving the list of instruction content in response to inputting the instruction topic into a trained instruction topic machine learning module (136) or rule module.

14. A method of medical communication, wherein the method comprises:
- receiving (204) a list of instruction content (136);
- receiving (206) initial subject data (134) from a subject data collection system (114), wherein the subject data collection system configured for collecting subject data descriptive of a subject (112); an
- determining (208) the instruction type using the initial subject data;
wherein execution of the machine executable instructions causes the computational system to repeatedly:
- retrieving (210) a next instruction section (140) from the instruction database by querying an instruction database with the instruction type and a next instruction content selected from the list of instruction content, wherein the instruction database comprises instruction sections indexed by instruction type and instruction content;
- presenting (212) the next instruction section using a presentation system (118), wherein the presentation system configured for displaying instructions to the subject; and
- adjusting (214) the instruction type using updated subject data (144) if the updated subject data is received from the subject data collection system.

15. A computer program comprising machine executable instructions (130), wherein execution of the machine executable instructions causes a computational system to:
- receive (204) a list of instruction content (136);
- receive (206) initial subject data (134) from a subject data collection system (114), wherein the subject data collection system configured for collecting subject data descriptive of a subject (112); and
- determine (208) the instruction type using the initial subject data;
wherein execution of the machine executable instructions causes the computational system to repeatedly:
- retrieve (210) a next instruction section (140) from the instruction database by querying an instruction database (132) with the instruction type and a next instruction content (142) selected from the list of instruction content, wherein the instruction database comprises instruction sections indexed by instruction type and the next instruction content;
- present (212) the next instruction section using a presentation system (118), wherein the presentation system configured for displaying instructions to the subject; and
- adjust (214) the instruction type using updated subject data (114) if the updated subject data is received from the subject data collection system.
